# EUROPÄISCHE PATENTSCHRIFT

(11) **EP 3 638 657 B1**
(45) Veröffentlichungstag und Bekanntmachung des Hinweises auf die Patenterteilung: **20.07.2022**
(21) Anmeldenummer: 18730753.3
(22) Anmeldetag: 11.06.2018
(51) Int. Cl.: C07D 333/76, C09K 19/34

(54) **DIBENZOFURAN- UND DIBENZOTHIOPHENDERIVATE**
DIBENZOFURAN DERIVATIVES AND DIBENZOTHIOPHENE DERIVATIVES
DÉRIVÉS DE DIBENZOFURANE ET DE DIBENZOTHIOPHÈNE

(30) Priorität: 14.06.2017 DE 102017005644
(43) Veröffentlichungstag der Anmeldung: 22.04.2020
(73) Patentinhaber: Merck Patent GmbH, 64293 Darmstadt (DE)
(72) Erfinder: LIETZAU, Lars, 64380 Rossdorf (DE); GOETZ, Achim, 64665 Alsbach-Haehnlein (DE); HIRSCHMANN, Harald, 64291 Darmstadt (DE); ENGEL, Martin, 64291 Darmstadt (DE)
(86) Internationale Anmeldenummer: PCT/EP2018/065277
(87) Internationale Veröffentlichungsnummer: WO 2018/228968

(56) Entgegenhaltungen:
- WO-A1-2009/091884
- DE-A1-102015 004 271

## Beschreibung

Die Erfindung betrifft Dibenzofuran und Dibenzothiophenderivate, deren Verwendung in Flüssigkristallmischungen, insbesondere in Flüssigkristallmischungen mit negativer dielektrischer Anisotropie sowie die Verwendung dieser Flüssigkristallmischungen in Flüssigkristallanzeigen vom VA-, PS-VA-, IPS-, PS-IPS- oder FFS-Typ.

Flüssigkristalle haben ein breites Anwendungsfeld gefunden, seitdem vor etwa 30 Jahren die ersten kommerziell anwendbaren flüssigkristallinen Verbindungen gefunden wurden. Bekannte Anwendungsgebiete für herkömmliche Mischungen sind insbesondere Anzeigen für Uhren und Taschenrechner sowie große Anzeigetafeln, wie sie in Bahnhöfen, Flughäfen und Sportarenen verwendet werden. Weitere Anwendungsgebiete sind Displays von tragbaren und stationären Computern, Navigationssystemen und Videoapplikationen. Insbesondere für die zuletzt genannten Anwendungen werden hohe Anforderungen an Schaltzeiten und den Kontrast der Abbildungen gestellt.

Die räumliche Ordnung der Moleküle in einem Flüssigkristall bewirkt, daß viele seiner Eigenschaften richtungsabhängig sind. Von Bedeutung für den Einsatz in Flüssigkristallanzeigen sind dabei insbesondere die Anisotropien im optischen, dielektrischen und elasto-mechanischen Verhalten. Je nachdem, ob die Moleküle mit ihren Längsachsen senkrecht oder parallel zu den beiden Platten eines Kondensators orientiert sind, hat dieser eine andere Kapazität; die Dielektrizitätskonstante ε des flüssigkristallinen Mediums ist also für die beiden Orientierungen verschieden groß. Substanzen, deren Dielektrizitätskonstante bei senkrechter Orientierung der Moleküllängsachsen zu den Kondensatorplatten größer ist als bei paralleler Anordnung, werden als dielektrisch positiv bezeichnet. Mit anderen Worten: Ist die Dielektrizitätskonstante ε|| parallel zu den Moleküllängsachsen größer als die Dielektrizitätskonstante ε⊥ senkrecht zu den Moleküllängsachsen, so ist die dielektrische Anisotropie Δε = ε|| - ε⊥ größer null. Die meisten Flüssigkristalle, die in herkömmlichen Displays Verwendung finden, fallen in diese Gruppe.

Für die dielektrische Anisotropie spielen sowohl die Polarisierbarkeit des Moleküls als auch permanente Dipolmomente eine Rolle. Beim Anlegen einer Spannung an das Display richtet sich die Längsachse der Moleküle so aus, daß die größere der dielektrischen Konstanten wirksam wird. Die Stärke der Wechselwirkung mit dem elektrischen Feld hängt dabei von der Differenz der beiden Konstanten ab.

Bei den in herkömmlichen Flüssigkristallanzeigen verwendeten flüssigkristallinen Molekülen ist das entlang der Moleküllängsachse orientierte Dipolmoment größer als das senkrecht zur Moleküllängsachse orientierte Dipolmoment.

Mit Flüssigkristallen, bei denen das größere Dipolmoment parallel zur Längsachse des Moleküls orientiert ist, sind bereits sehr leistungsfähige Displays entwickelt worden. Dabei kommen meist Mischungen von 5 bis 20 Komponenten zum Einsatz, um einen ausreichend breiten Temperaturbereich der Mesophase sowie kurze Schaltzeiten und niedrige Schwellenspannungen anzustreben. Schwierigkeiten bereitet jedoch noch die starke Blickwinkelabhängigkeit bei Flüssigkristallanzeigen, wie sie beispielsweise für Laptops verwendet werden. Die beste Abbildungsqualität lässt sich erreichen, wenn die Fläche des Displays senkrecht zur Blickrichtung des Betrachters steht. Wird das Display relativ zur Betrachtungsrichtung gekippt, verschlechtert sich die Abbildungsqualität unter Umständen drastisch. Für einen höheren Komfort ist man bemüht, den Winkel, um den das Display von der Blickrichtung eines Betrachters ohne wesentliche Minderung der Abbildungsqualität gekippt werden kann, möglichst groß zu gestalten. In jüngerer Zeit sind Versuche unternommen worden, zur Verbesserung der Blickwinkelabhängigkeit flüssigkristalline Verbindungen einzusetzen, deren Dipolmoment senkrecht zur Moleküllängsachse größer ist als parallel zur Längsachse des Moleküls. Die dielektrische Anisotropie Δε ist in diesem Fall negativ. Im feldfreien Zustand werden diese Moleküle mit ihrer Längsachse senkrecht zur Glasfläche des Displays orientiert. Durch Anlegen eines elektrischen Feldes orientieren sie sich mehr oder weniger parallel zu den Glasflächen. Auf diese Weise konnte eine Verbesserung der Blickwinkelabhängigkeit erreicht werden. Derartige Displays werden als VA-TFT-Displays bezeichnet (abgeleitet aus dem Englischen: "vertically aligned").

Weitere FK-Anzeigemodi, die neben der TV-Anwendung besonders auch für kleine und mittelgroße FK-Anzeigen zur Anwendung in tragbaren Geräten wie beispielsweise Tablet-PC oder sog. Smart-Phones zu Anwendung kommen, sind der IPS- und der FFS-Modus (engl. fringe field switching), in denen FK-Medien mit sowohl negativer als auch positiver dielektrischer Anisotropie zum Einsatz kommen.

FFS -Anzeigen, die auf FK-Medien mit negative dielektrischer Anisotropie basieren, wurden in S.H. Lee et al., Appl. Phys. Lett. 73(20), 1998, 2882-2883 und S.H. Lee et al., Liquid Crystals 39(9), 2012, 1141-1148 beschrieben.

Die Entwicklung auf dem Gebiet der flüssigkristallinen Materialien ist bei weitem noch nicht abgeschlossen. Zur Verbesserung der Eigenschaften flüssigkristalliner Anzeigeelemente ist man ständig bemüht, neue Verbindungen zu entwickeln, die eine Optimierung derartiger Displays ermöglichen.

Aus dem Stand der Technik sind VA-Materialien bekannt, die vom Dibenzofuran oder vom Dibenzothiophen abgeleitet sind.

In WO 02/055463 sind Verbindungen der Formel 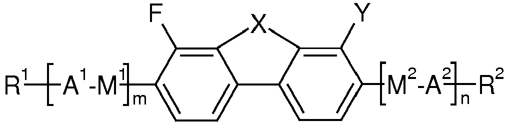 offenbart, worin X u.a. O oder S, Y F, R¹ und R² Alkyl oder Alkoxy bedeuten können und die übrigen Parameter die dort angegebene Bedeutung haben. Die dort beschriebenen Verbindungen besitzen neg. dielektrische Anisotropie, sind allerdings für ferroelektrische FK-Mischungen entwickelt worden und es werden keine Werte für die dielektrischen Anisotropien der Einzelsubstanzen beschrieben.

Aus der WO 2009/091884 sind Verbindungen grundsätzlich bekannt, die einen fünfgliedrigen Carbocyclus enthalten, u.a. die Verbindung der folgenden Formel: 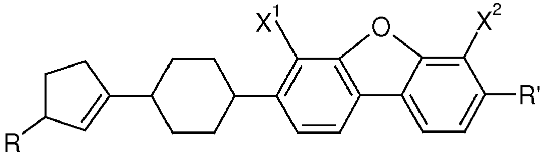 worin X¹ und X² H oder F, R Alkyl und R' Alkyl oder Alkoxy bedeuten. Allerdings wird kein konkretes Beispiel dieser Substanzklasse beschrieben.

Eine Aufgabe der vorliegenden Erfindung ist es, Verbindungen mit vorteilhaften Eigenschaften für den Einsatz in flüssigkristallinen Medien zur Verfügung zu stellen. Bevorzugt sollten sie über eine negative dielektrische Anisotropie verfügen, was sie besonders für den Einsatz in flüssigkristallinen Medien für VA- oder FFS-Displays geeignet macht. Unabhängig von der dem Displaytyp entsprechenden dielektrischen Anisotropie sind Verbindungen gewünscht, die eine günstige Kombination der anwendungstechnischen Parameter aufweisen. Unter diesen gleichzeitig zu optimierenden Parametern sind vor allem ein hoher Klärpunkt, eine geringe Rotationsviskosität und eine geeignete optische Anisotropie im Anwendungsintervall zu nennen, sowie die Eigenschaften, die zur Erzielung von Mischungen mit den gewünschten flüssigkristallinen Phasen über einen breiten Temperaturbereich dienen (niedriger Schmelzpunkt, gute Mischbarkeit mit anderen flüssigkristallinen Komponenten der gewünschten Art und gute Löslichkeit bei hoher Polarität).

Die Erfindung betrifft Verbindungen der Formel I 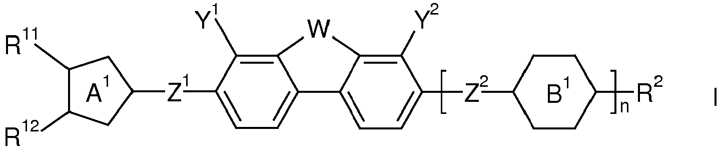 worin
- W: -O- oder -S-,
- Y¹ und Y²: unabhängig voneinander H, F, Cl, CN oder CF₃,
- R¹¹ und R¹²: unabhängig voneinander, H, einen unsubstituierten, einen einfach durch CN oder CF₃ oder mindestens einfach durch Halogen substituierten Alkyl- oder Alkenylrest mit bis zu 15 C-Atomen, wobei in diesen Resten auch eine oder mehrere CH₂-Gruppen durch -O-, -S-, -C=C-, -CF₂O-, -OCF₂-, -OC-O- oder -O-CO- so ersetzt sein können, daß O-Atome nicht direkt miteinander verknüpft sind,
- R²: einen unsubstituierten, einen einfach durch CN oder CF₃ oder mindestens einfach durch Halogen substituierten Alkyl- oder Alkenylrest mit bis zu 15 C-Atomen, wobei in diesen Resten auch eine oder mehrere CH₂-Gruppen durch -O-, -S-, -C=C-, -CF₂O-, -OCF₂-, -OC-O- oder-O-CO- so ersetzt sein können, daß O-Atome nicht direkt miteinander verknüpft sind, ferner H, Halogen, CN, SCN oder SF₅.
- A¹: einen 1,3-Cyclopentylenrest, der einfach ungesättigt sein kann und der ein- oder mehrfach durch eine Gruppe L substituiert sein kann,
- B¹: bei jedem Auftreten gleich oder verschieden,
a) einen 1,4-Phenylenrest, worin eine oder zwei CH-Gruppen durch N ersetzt sein können,
b) einen 1,4-Cyclohexenylen- oder 1,4-Cyclohexylenrest, worin eine oder zwei nicht benachbarte CH₂-Gruppen durch -O- oder -S- ersetzt sein können, einen Cyclobutan-1,3-diylrest,
c) einen Rest aus der Gruppe 1,4-Bicyclo[2,2,2]-octylen-, spiro[3.3]heptan-2,6-diyl, Naphthalin-2,6-diyl, Decahydronaphthalin-2,6-diyl, 1,2,3,4-Tetrahydro-naphthalin-2,6-diyl, Thiophen-2,5-diyl, wobei die Gruppen a), b) und c) ein oder mehrfach durch eine Gruppe L substituiert sein können.
- L: bei jedem Auftreten unabhängig F, Cl, CN, SCN, SF₅ oder geradkettiges oder verzweigtes, jeweils optional fluoriertes, Alkyl, Alkoxy, Alkylcarbonyl, Alkoxycarbonyl, Alkylcarbonyloxy oder Alkoxycarbonyloxy mit 1 bis 12 C-Atomen,
- Z¹ und Z²: jeweils unabhängig voneinander eine Einfachbindung, -CF₂O-, -OCF₂-, -CF₂S-, -SCF₂-, -CH₂CH₂-, -CF₂CF₂-, -CF₂CH₂-, -CH₂CF₂-, -CHF-CHF-, -C(O)O-, -OC(O)-, -CH₂O-, -OCH₂-, -CH₂S-, -SCH₂-, -CF=CH-, -CH=CF-, -CF=CF-, -CH=CH- oder -C≡C- ,
- n: 0 bedeuten.

Die Verbindungen besitzen eine ausgeprägt negative dielektrische Anisotropie (Δε) und eignen sich daher insbesondere für eine Verwendung in Flüssigkristallmischungen für VA-TFT-Displays oder Displays vom IPS oder FFS-Typ. Für Anwendungen kann ein möglichst stark negatives Δε vorteilhaft sein. Die erfindungsgemäßen Verbindungen besitzen eine dielektrische Anisotropie Δε von vorzugsweise -4 oder weniger, bevorzugt von -6 oder weniger und ganz besonders bevorzugt von -8 oder weniger. Sie zeigen eine gute Mischbarkeit mit den üblichen, in Flüssigkristallmischungen für Displays verwendeten Substanzen, d. h. sie besitzen eine gute Löslichkeit darin. Die Rotationsviskositäten der Verbindungen und der resultierenden flüssigkristallinen Mischungen sind vorteilhaft klein und die jeweiligen Klärpunkte gleichzeitig vorteilhaft hoch. Auch die weiteren physikalischen, physikochemischen beziehungsweise elektrooptischen Parameter der erfindungsgemäßen Verbindungen sind für den Einsatz der Verbindungen in flüssigkristallinen Medien von Vorteil. Die flüssigkristallinen Medien, die diese Verbindungen enthalten, weisen insbesondere eine ausreichende Breite der nematischen Phase und eine gute Tieftemperatur- und Langzeitstabilität sowie ausreichend hohe Klärpunkte auf. Die niedrigen Schmelzpunkte der erfindungsgemäßen Verbindungen geben einen Hinweis auf das vorteilhafte Mischungsverhalten. Ferner weisen die erfindungsgemäßen Verbindungen der Formel 1 insbesondere für die Verwendung in VA-TFT-Displays oder Displays vom IPS-oder FFS-Typ geeignete Werte der optischen Anisotropie Δn auf. Bevorzugt besitzen die erfindungsgemäßen Verbindungen ein Δn von größer als 0,15 und kleiner als 0,25. Außerdem sind die Verbindungen relativ einfach herzustellen. Die ausgewogene Kombination dieser vorteilhaften Eigenschaften stellt eine signifikante Bereicherung der für Mischungen mit negativer dielektrischer Anisotropie verfügbaren Mischungskomponenten dar.

Sofern R¹¹, R¹² und R² in Formel I jeweils unabhängig voneinander einen Alkylrest darstellen, sind diese geradkettig oder verzweigt. Vorzugsweise ist jeder dieser Reste geradkettig, und hat soweit nicht anders angegeben 1, 2, 3, 4, 5, 6 oder 7 C-Atome und ist demnach vorzugsweise Methyl, Ethyl, Propyl, Butyl, Pentyl, Hexyl oder Heptyl.

Sofern R¹¹, R¹² und R² in Formel I jeweils unabhängig voneinander einen Alkoxyrest darstellen, sind diese geradkettig oder verzweigt. Vorzugsweise ist jeder dieser Reste geradkettig, und hat soweit nicht anders angegeben 1, 2, 3, 4, 5, 6 oder 7 C-Atome und ist demnach vorzugsweise Methoxy, Ethoxy, Propoxy, Butoxy, Pentoxy, Hexoxy oder Heptoxy.

R¹¹, R¹² und R² in Formel I können ferner jeweils unabhängig voneinander ein Alkenylrest mit 2 bis 15 C-Atomen sein, der geradkettig oder verzweigt ist und wenigstens eine C-C-Doppelbindung aufweist. Vorzugsweise ist er geradkettig und hat 2 bis 7 C-Atome. Er ist demnach vorzugsweise Vinyl, Prop-1- oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl. Sind die beiden C-Atome der C-C-Doppelbindung substituiert, kann der Alkenylrest als E- und/oder Z-Isomer (trans/cis) vorliegen. Im Allgemeinen sind die jeweiligen E-Isomere bevorzugt. Unter den Alkenylresten sind besonders bevorzugt Prop-2-enyl, 2- oder But-3-enyl, und 3- oder Pent-4-enyl.

R¹¹, R¹² und R² in Formel I können unabhängig voneinander auch ein Alkinylrest mit 2 bis 15 C-Atomen sein, der geradkettig oder verzweigt ist und wenigstens eine C-C-Dreifachbindung aufweist. Bevorzugt ist 1- oder 2 Propinyl und 1-, 2- oder 3- Propinyl.

Falls R² für einen polaren Rest steht, so ist dieser ausgewählt aus Halogen, CN, SCN, SF₅, ein-, zwei- oder mehrfach fluoriertem Alkyl, Alkenyl, Alkoxy oder Alkenyloxy mit bis zu 15 C-Atomen, bevorzugt F, Cl, CN, SCN, SF₅, CF₂H, CF₃, OCF₂H, OCF₃ oder -OCH=CF₂.

Die Gruppe Z¹ bedeutet bevorzugt eine Einfachbindung, -CH₂O-, -OCH₂-, -CF₂O - oder -OCF₂, besonders bevorzugt eine Einfachbindung.

Die Gruppe Z² bedeutet bevorzugt eine Einfachbindung, -CH₂O-, -OCH₂-, -CF₂O - oder -OCF₂, besonders bevorzugt eine Einfachbindung.

Bevorzugt ist A¹ ausgewählt aus den Ringelementen der Formeln 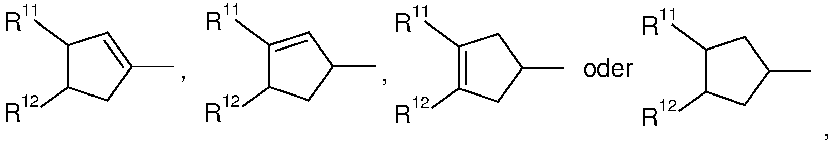 besonders bevorzugt 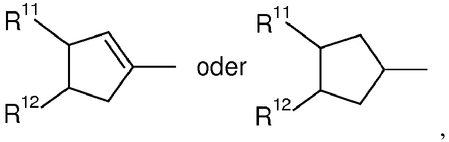 ganz besonders bevorzugt

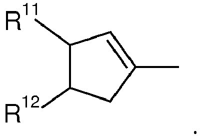

Darin bedeuten R¹¹ und R¹² unabhängig voneinander bevorzugt H oder Alkyl mit 1 bis 7 C-Atomen.

Besonders bevorzugt bedeutet mindestens eine der Gruppen R¹¹ und R¹² H.

In einer ganz besonders bevorzugten Ausführungsform bedeuten R¹¹ und R¹² beide H.

In einer bevorzugten Ausführungsform der vorliegenden Erfindung bedeutet W-S-.

Halogen bedeutet im Zusammenhang der vorliegenden Erfindung Fluor, Chlor, Brom und lod, insbesondere Fluor und Chlor.

Die Gruppe L bedeutet bevorzugt F, Cl, -CF₃ oder eine Alkyl- oder Alkoxygruppe mit 1, 2 oder 3 Kohlenstoffatomen.

Bevorzugt ist mindestens einer der Reste Y¹ und Y² in Formel I von H verschieden.

Besonders bevorzugt bedeuten Y¹ und Y² in Formel I beide F.

Bevorzugt bedeutet n 0 oder 1, besonders bevorzugt 0.

Die Verbindungen der Formel I sind bevorzugt ausgewählt aus der Gruppe der Verbindungen der Formeln I-1 und I-2. 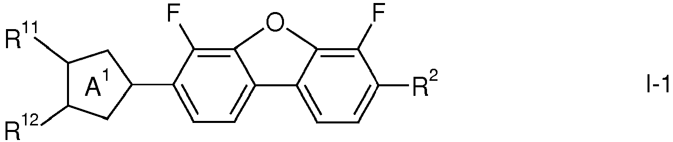 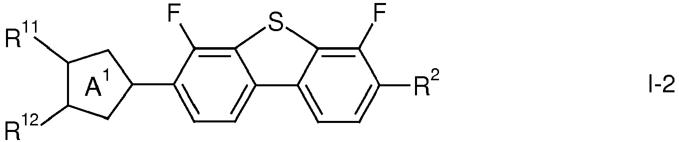 worin R¹¹, R¹² und R² die oben angegebenen Bedeutungen haben.

Ganz besonders bevorzugt sind die Verbindungen der Formel I-2. Die Verbindungen der Formeln I-1 und I-2 sind bevorzugt ausgewählt aus der Gruppe der Verbindungen der folgenden Unterformel: 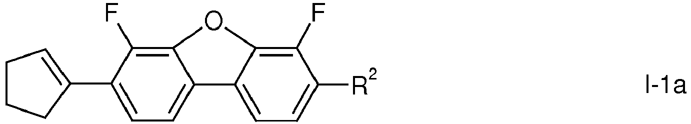 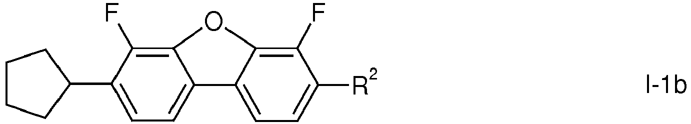 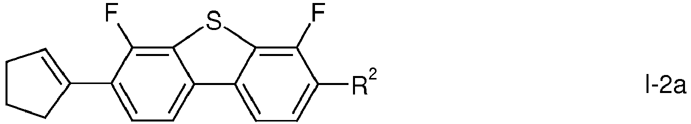 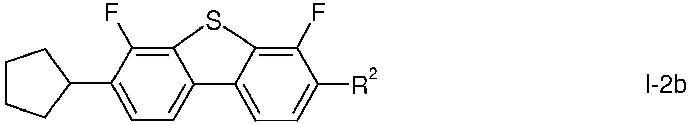 worin R² die oben angegebene Bedeutung hat.

In einer ersten bevorzugten Ausführungsform bedeutet R² in den Formeln I-1a, I-1b, I-2a und I-2b Methyl, Ethyl, n-Propyl, n-Butyl, n-Pentyl, n-Hexyl oder n-Heptyl.

In einer zweiten bevorzugten Ausführungsform bedeutet R² in den Formeln I-1a, I-1b, I-2a und I-2b Methoxy, Ethoxy, n-Propoxy, n-Butoxy, n-Pentoxy, n-Hexoxy oder n-Heptoxy.

In einer dritten bevorzugten Ausführungsform bedeutet R² in den Formeln I-1a, I-1b, I-2a und I-2b F, Cl, CN, SCN, SF₅, CF₂H, CF₃, OCF₂H, OCF₃ oder -OCH=CF₂, besonders bevorzugt F, CF₃ oder OCF₃.

Sofern Reste oder Substituenten der erfindungsgemäßen Verbindungen beziehungsweise die erfindungsgemäßen Verbindungen selbst als optisch aktive oder stereoisomere Reste, Substituenten beziehungsweise Verbindungen vorliegen, weil sie beispielsweise ein asymmetrisches Zentrum aufweisen, so sind diese von der vorliegenden Erfindung mit umfaßt. Dabei ist es selbstverständlich, daß die erfindungsgemäßen Verbindungen der allgemeinen Formel I in isomerenreiner Form, zum Beispiel als reine Enantiomeren, Diastereomeren, E- beziehungsweise Z-Isomeren, transbeziehungsweise cis-Isomeren, oder als Gemisch mehrerer Isomeren in jedem beliebigen Verhältnis, zum Beispiel als Racemat, E-/Z-Isomerengemisch oder als cis/trans-Isomerengemisch, vorliegen können.

Der 1,4-substituierte Cyclohexylring der Formel 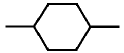 oder -Cycist in den offenbarten Verbindungen für flüssigkristalline Medien bevorzugt trans-konfiguriert, d.h. die beiden Substituenten befindend sich in der thermodynamisch bevorzugten Sesselkonformation beide in äquatorialer Position.

Die Verbindungen der allgemeinen Formel I können nach an sich bekannten Methoden dargestellt werden, wie sie in der Literatur (z.B. in den Standardwerken wie Houben-Weyl, Methoden der organischen Chemie, Georg-Thieme-Verlag, Stuttgart) beschrieben sind und zwar unter Reaktionsbedingungen, die für die genannten Umsetzungen bekannt und geeignet sind. Dabei kann man von an sich bekannten, hier nicht näher erwähnten Varianten Gebrauch machen.

Die Ausgangsstoffe können gegebenenfalls auch *in situ* gebildet werden, derart, daß man sie aus dem Reaktionsgemisch nicht isoliert, sondern sofort weiter zu den Verbindungen der allgemeinen Formel I umsetzt.

Die Synthesen erfindungsgemäßer Verbindungen der allgemeinen Formel I werden in den Beispielen exemplarisch beschrieben. Die Ausgangssubstanzen sind nach allgemein zugänglichen Literaturvorschriften oder käuflich zu erhalten.

Ein besonders bevorzugter Syntheseweg ist in Schema 1 aufgezeigt:

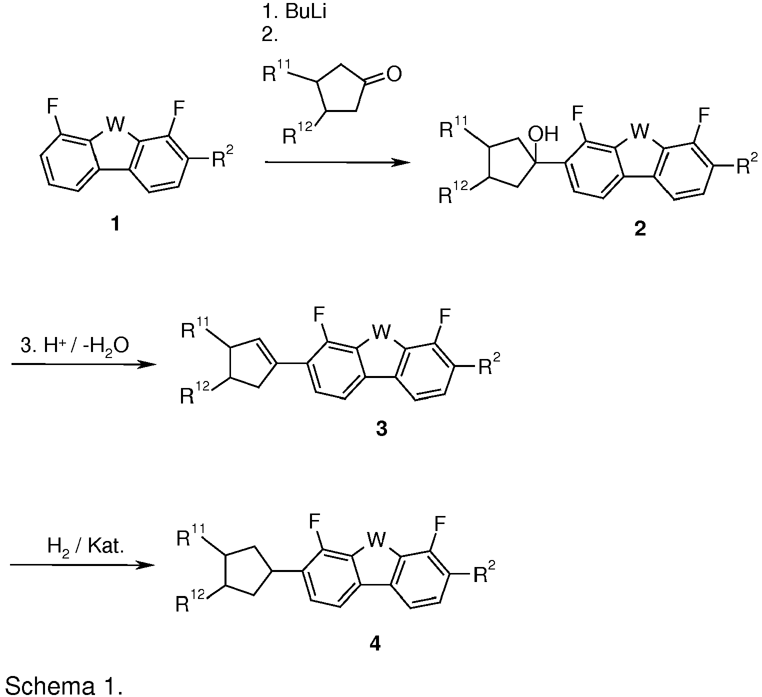

Geeignete Vorstufen (1) sind in WO 02/055463 und DE 10 2015 002298 A1 (für W = O, R² = Alkyl, Alkoxy), in der EP 2 937 342 A1 (für W = S, R² = Alkyl, Alkoxy) sowie in der EP 3 085 753 A1 (für W = O, S, R² = F, Cl, CN, SCN, SF₅, CF₂H, CF₃, OCF₂H, OCF₃ oder -OCH=CF₂) beschrieben. Aus den Verbindungen 1 werden nach Ortho-Metallierung, Addition an entsprechend substituierte Cyclopentanonderivate zu den Alkoholen 2 und nachfolgende Wasserabspaltung die erfindungsgemäßen Produkte 3 erhalten, die ggf. weiter durch katalytische Hydrierung zu den erfindungsgemäßen Verbindungen 4 umgesetzt werden können. Bevorzugte Bedingungen für die Metallierung sind die Umsetzung mit einem Lithiumalkyl wie n-BuLi , in THF, bei ca. -70°C, dann Zugabe des Ketons.

Cyclopentanon sowie Alkyl- und Dialkylcyclopentanonderivate sind kommerziell erhältlich, literaturbekannt oder können in zu den literaturbekannten Synthesen analoger Weise hergestellt werden, sowohl als Racemat als auch in optisch aktiver Form. Vorzugsweise werden gemäß der vorliegenden Erfindung alkylsubstituierte Cyclopentanone als Racemate eingesetzt.

Beispiele für bevorzugte Cyclopentanonderivate sind:

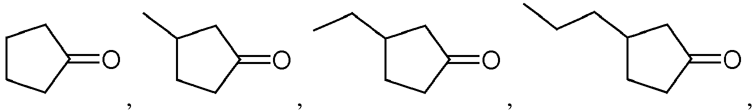

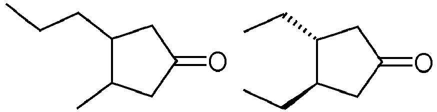

Gegenstand der Erfindung sind weiterhin Verbindungen der Formel II 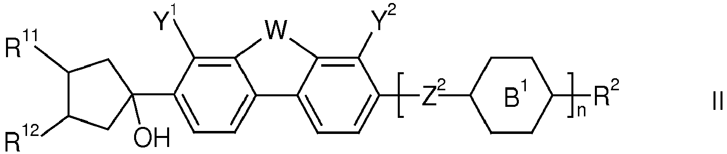 worin R¹¹, R¹², Y¹, Y², W, Z², B¹, R² und n die für Formel I angegebenen Bedeutungen haben.

Verbindungen der Formel II werden gemäß der in Schema 1 und in den Ausführungsbeispielen angegebenen Synthesen hergestellt und können durch Eliminierung von Wasser in die Verbindungen der Formel I übergeführt werden.

Verfahren zur Eliminierung von Wasser aus Alkoholen sind dem Fachmann bekannt und beispielsweise in Houben-Weyl, Methoden der organischen Chemie, Georg-Thieme-Verlag, Stuttgart beschrieben.

Das Verfahren und die anschließende Aufarbeitung des Reaktionsgemisches kann grundsätzlich als Batch-Reaktion oder in kontinuierlicher Reaktionsweise durchgeführt werden. Die kontinuierliche Reaktionsweise umfaßt z. B. die Reaktion in einem kontinuierlichen Rührkesselreaktor, einer Rührkesselkaskade, einem Schlaufen- oder Querstromreaktor, einem Strömungsrohr oder in einem Mikroreaktor. Die Aufarbeitung der Reaktionsgemische erfolgt wahlweise, je nach Bedarf, durch Filtration über feste Phasen, Chromatographie, Separation zwischen unmischbaren Phasen (z. B. Extraktion), Adsorption an festen Trägern, Abdestillieren von Lösungsmitteln und/oder azeotropen Gemischen, selektive Destillation, Sublimation, Kristallisation, Co-Kristallisation oder durch Nanofiltration an Membranen.

Wie bereits erwähnt, können die Verbindungen der allgemeinen Formel I in flüssigkristallinen Medien verwendet werden. Gegenstand der vorliegenden Erfindung ist daher auch ein flüssigkristallines Medium mit mindestens zwei flüssigkristallinen Verbindungen, enthaltend mindestens eine Verbindung der allgemeinen Formel I.

Gegenstand der vorliegenden Erfindung sind auch flüssigkristalline Medien enthaltend neben einer oder mehreren erfindungsgemäßen Verbindungen der Formel I als weitere Bestandteile 2 bis 40, vorzugsweise 4 bis 30 Komponenten. Besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, 1,3-Dioxane, 2,5-Tetrahydropyrane, Phenyl- oder Cyclohexylbenzoate, Cyclohexancarbonsäurephenyl- oder-cyclohexylester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Biscyclohexylbenzole, 4',4'-Biscyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenylcyclohexyl)ethane, 1-Cyclohexyl-2-biphenylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch einfach oder mehrfach fluoriert sein.

Die wichtigsten als weitere Bestandteile erfindungsgemäßer Medien in Frage kommenden Verbindungen lassen sich durch die Formeln (II), (III), (IV), (V) und (VI) charakterisieren:

R'-L-E-R" (II)

R'-L-COO-E-R" (III)

R'-L-OOC-E-R" (IV)

R'-L-CH₂CH₂-E-R" (V)

R'-L-CF₂O-E-R" (VI)

In den Formeln (II), (III), (IV), (V) und (VI) bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -Thp-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl, Thp Tetrahydropyran-2,5-diyl und G 2-(trans-1,4-Cyclohexyl)-ethyl, Pyrimidin-2,5-diyl, Pyridin-2,5-diyl, 1,3-Dioxan-2,5-diyl oder Tetrahydropyran-2,5-diyl bedeuten.

Vorzugsweise ist einer der Reste L und E Cyc oder Phe. E ist vorzugsweise Cyc, Phe oder Phe-Cyc. Vorzugsweise enthalten die erfindungsgemäßen Medien eine oder mehrere Komponenten ausgewählt aus den Verbindungen der Formeln (II), (III), (IV), (V) und (VI), worin L und E ausgewählt sind aus der Gruppe Cyc und Phe und gleichzeitig eine oder mehrere Komponenten ausgewählt aus den Verbindungen der Formeln (II), (III), (IV), (V) und (VI), worin einer der Reste L und E ausgewählt ist aus der Gruppe Cyc und Phe und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln (II), (III), (IV), (V) und (VI), worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln (II), (III), (IV), (V) und (VI) jeweils unabhängig voneinander Alkyl, Alkenyl, Alkoxy, Alkoxyalkyl (Oxaalkyl), Alkenyloxy oder Alkanoyloxy mit bis zu 8 C-Atomen. Im folgenden wird diese kleinere Untergruppe Gruppe A genannt und die Verbindungen werden mit den Teilformeln (IIa), (IIIa), (IVa), (Va) und (VIa) bezeichnet. Bei den meisten dieser Verbindungen sind R' und R" voneinander verschieden, wobei einer dieser Reste meist Alkyl, Alkenyl, Alkoxy oder Alkoxyalkyl (Oxaalkyl) ist.

In einer anderen als Gruppe B bezeichneten kleineren Untergruppe der Verbindungen der Formeln (II), (III), (IV), (V) und (VI) bedeutet E

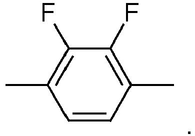

In den Verbindungen der Gruppe B, die mit den Teilformeln (IIb), (IIIb), (IVb), (Vb) und (VIb) bezeichnet werden, haben R' und R" die bei den Verbindungen der Teilformeln (IIa) bis (VIa) angegebene Bedeutung und sind vorzugsweise Alkyl, Alkenyl, Alkoxy oder Alkoxyalkyl (Oxaalkyl).

In einer weiteren kleineren Untergruppe der Verbindungen der Formeln (II), (III), (IV), (V) und (VI) bedeutet R" -CN. Diese Untergruppe wird im Folgenden als Gruppe C bezeichnet und die Verbindungen dieser Untergruppe werden entsprechend mit Teilformeln (IIc), (IIIc), (IVc), (Vc) und (Vlc) beschrieben. In den Verbindungen der Teilformeln (IIc), (IIIc), (IVc), (Vc) und (Vlc) hat R' die bei den Verbindungen der Teilformeln (IIa) bis (VIa) angegebene Bedeutung und ist vorzugsweise Alkyl, Alkenyl, Alkoxy oder Alkoxyalkyl (Oxaalkyl).

Neben den bevorzugten Verbindungen der Gruppen A, B und C sind auch andere Verbindungen der Formeln (II), (III), (IV), (V) und (VI) mit anderen Varianten der vorgesehenen Substituenten gebräuchlich. All diese Substanzen sind nach literaturbekannten Methoden oder in Analogie dazu erhältlich.

Die erfindungsgemäßen Medien enthalten neben den erfindungsgemäßen Verbindungen der allgemeinen Formel I vorzugsweise eine oder mehrere Verbindungen aus den Gruppen A, B und/oder C. Die Massenanteile der Verbindungen aus diesen Gruppen an den erfindungsgemäßen Medien betragen:
Gruppe A:
   0 bis 90 %, vorzugsweise 20 bis 90 %, insbesondere 30 bis 90 %.
Gruppe B:
   0 bis 80 %, vorzugsweise 10 bis 80 %, insbesondere 10 bis 70 %.
Gruppe C:
   0 bis 80 %, vorzugsweise 5 bis 80 %, insbesondere 5 bis 50 %.

Die erfindungsgemäßen Medien enthalten vorzugsweise 1 bis 40 %, besonders bevorzugt 5 bis 30 % an den erfindungsgemäßen Verbindungen der Formel I. Die Medien enthalten vorzugsweise eine, zwei, drei, vier oder fünf erfindungsgemäße Verbindungen der Formel I.

Die Herstellung der erfindungsgemäßen Medien erfolgt in an sich üblicher Weise. In der Regel werden die Komponenten ineinander gelöst, vorzugsweise bei erhöhter Temperatur. Durch geeignete Zusätze können die flüssigkristallinen Phasen der vorliegenden Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die Verbindungen der Formel I eignen sich wegen ihres negativen Δε insbesondere für eine Verwendung in VA-TFT-Displays.

Gegenstand der vorliegenden Erfindung sind daher auch elektrooptische Anzeigeelemente, enthaltend ein erfindungsgemäßes flüssigkristallines Medium. Bevorzugt ist das Anzeigeelement ein VA-TFT-Anzeigeelement (VA: vertical alignment; TFT: thin film transistor).

Weitere Kombinationen der Ausführungsformen und Varianten der Erfindung gemäß der Beschreibung ergeben sich aus den Ansprüchen.

Weitere Ausführungsformen der vorliegenden Erfindung ergeben sich aus den Ansprüchen und aus Kombinationen zweier oder mehrerer dieser Ansprüche.

Die Erfindung wird im Folgenden anhand von Ausführungsbeispielen näher erläutert, ohne dadurch jedoch eingeschränkt werden zu sollen. Der Fachmann wird in der Lage sein, den Beispielen Details zur Durchführung zu entnehmen, die in der allgemeinen Beschreibung nicht im Einzelnen aufgeführt sind, sie nach allgemeinen Fachkenntnissen zu verallgemeinern und auf seine spezielle Problemstellung anzuwenden.

Neben den üblichen und wohlbekannten Abkürzungen werden folgende Abkürzungen verwendet:
K: Kristalline Phase; N: Nematische Phase; Sm: Smektische Phase;
I: Isotrope Phase. Die Zahlen zwischen diesen Symbolen geben die Übergangstemperaturen der betreffenden Substanz wieder. Temperaturangaben sind, soweit nichts anderes angegeben, in °C.

Die Bestimmung physikalischer, physikochemischer beziehungsweise elektrooptischer Parameter erfolgt nach allgemein bekannten Verfahren, wie sie unter anderem beschrieben sind in der Broschüre "Merck Liquid Crystals - Licristal® - Physical Properties of Liquid Crystals - Description of the Measurements Methods", 1998, Merck KGaA, Darmstadt.

Vor- und nachstehend bedeutet Δn die optische Anisotropie (589 nm, 20 °C) und Δε die dielektrische Anisotropie (1 kHz, 20 °C). Die dielektrische Anisotropie Δε wird bei 20°C und 1 kHz bestimmt. Die optische Anisotropie Δn wird bei 20°C und einer Wellenlänge von 589,3 nm bestimmt.

Die Δε- und Δn-Werte, der extrapolierte Klärpunkt (Klp.) sowie die Rotationsviskosität (γ₁) der erfindungsgemäßen Verbindungen werden durch lineare Extrapolation aus flüssigkristallinen Mischungen erhalten, die zu 5 bis 10 % aus der jeweiligen erfindungsgemäßen Verbindung und zu 90-95 % aus der kommerziell erhältlichen Flüssigkristallmischung ZLI-2857 (für Δε, Klp.) bzw. ZLI-4792 (für Δn, γ₁) bestehen (Mischungen Fa. Merck KGaA, Darmstadt).

Nachstehend bedeuten die Abkürzungen:
- RT: Raumtemperatur, ca. 20 °C
- Schmp.: Schmelzpunkt
- MTB: Methyl-tert.-butylether
- THF: Tetrahydrofuran
- EE: Essigester
- BuCl: n-Butylchlorid
- TsOH: Toluolsulfonsäure
- BuLi: n-Butyllithium

Die Synthese der Verbindung 1 ist in DE 10 2015 004271 A1 beschrieben.

Substanzbeispiel 1

### 1.1 1-(7-Ethoxy-4,6-difluor-dibenzothiophen-3-yl)cyclopentanol (3)

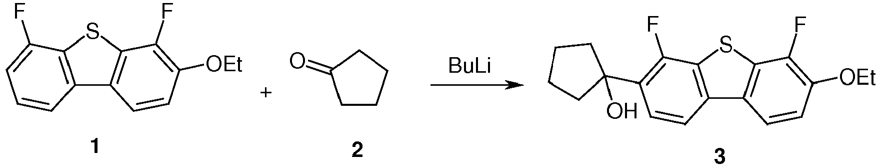

5.3 g (20 mmol) des Dibenzothiophens 1 werden in 50 ml THF gelöst und bei -70°C mit 16.9 ml Butyllithium (15%) in Hexan (26 mmol) versetzt. Nach 1h bei -70°C werden 2.3 g (30 mmol) Cyclopentanon (2), gelöst in 20 ml THF, hinzugefügt. Die Kühlung wird entfernt und bei -20°C wird der Ansatz mit Wasser und MTB-Ether versetzt. Die wässerige Phase wird mit MTB-Ether extrahiert, die vereinigten organischen Phasen über Natriumsulfat getrocknet, filtriert und eingeengt. Der Rückstand wird über Kieselgel (BuCl/EE) filtriert und die Produktfraktionen eingeengt. Der erhaltene Rückstand (2.5g (81%)) (3) wird ohne weitere Reinigung in der Folgestufe eingesetzt.

### 1.2 3-(Cyclopenten-1-yl)-7-ethoxy-4,6-difluordibenzothiophen (4)

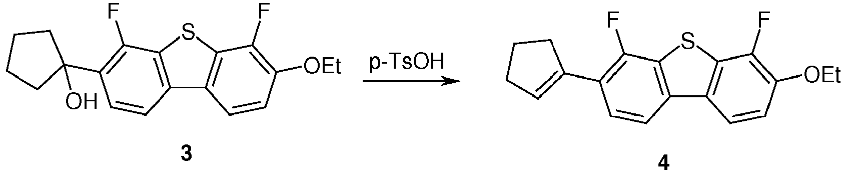

3.5 g (10 mmol) des Alkohols 3 werden in 80 ml Toluol gelöst, mit 200 mg p-TsOH versetzt und am Wasserabscheider zum Sieden erhitzt. Der abgekühlte Ansatz wird über Kieselgel filtriert und mit n-Heptan eluiert. Der nach Einengen des Filtrats erhaltene Rückstand wird aus Ethanol/Toluol kristallisiert. Man erhält 3-(Cyclopenten-1-yl)-7-ethoxy-4,6-difluordibenzothiophen als farblose Kristalle vom Schmp. 153 °C.
Δε = -8,24
Δn = 0,2815
γ₁ = 235 mPa s

### Beispiel 2: 3-(Cyclopentyl)-7-ethoxy-4,6-difluordibenzothiophen (4)

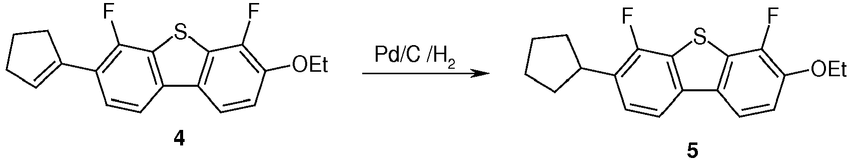

5,3 g (20 mmol) des Cyclopentens 4 werden in 55 ml THF gelöst und an Pd/C bis zum Stillstand hydriert. Die Lösung wird anschließend filtriert, eingeengt, der Rückstand mit n-Heptan/EE über Kieselgel filtriert und aus Ethanol und anschließend aus Heptan umkristallisiert. Man erhält 3-(Cyclopentyl)-7-ethoxy-4,6-difluordibenzothiophen als farblose Kristalle vom Schmp. 110 °C.
Δε = -8,41
Δn = 0,2035
γ₁ = 276 mPa s

## Patentansprüche

1. Verbindung der Formel I 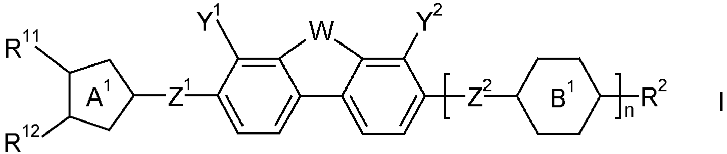 worin
W -O- oder -S-,
Y¹ und Y² unabhängig voneinander H, F, Cl, CN oder CF₃,
R¹¹ und R¹² unabhängig voneinander, H, einen unsubstituierten, einen einfach durch CN oder CF₃ oder mindestens einfach durch Halogen substituierten Alkyl- oder Alkenylrest mit bis zu 15 C-Atomen, wobei in diesen Resten auch eine oder mehrere CH₂-Gruppen durch -O-, -S-, -C=C-, -CF₂O-, -OCF₂-, -OC-O- oder -O-CO- so ersetzt sein können, daß O-Atome nicht direkt miteinander verknüpft sind,
R² einen unsubstituierten, einen einfach durch CN oder CF₃ oder mindestens einfach durch Halogen substituierten Alkyl- oder Alkenylrest mit bis zu 15 C-Atomen, wobei in diesen Resten auch eine oder mehrere CH₂-Gruppen durch -O-, -S-, -C=C-, -CF₂O-, -OCF₂-, -OC-O- oder-O-CO- so ersetzt sein können, daß O-Atome nicht direkt miteinander verknüpft sind, ferner H, Halogen, CN, SCN oder SF₅.
A¹ einen 1,3-Cyclopentylenrest, der einfach ungesättigt sein kann und der ein- oder mehrfach durch eine Gruppe L substituiert sein kann,
B¹ bei jedem Auftreten gleich oder verschieden,
a) einen 1,4-Phenylenrest, worin eine oder zwei CH-Gruppen durch N ersetzt sein können,
b) einen 1,4-Cyclohexenylen- oder 1,4-Cyclohexylenrest, worin eine oder zwei nicht benachbarte CH₂-Gruppen durch -O- oder -S- ersetzt sein können, einen Cyclobutan-1,3-diylrest,
c) einen Rest aus der Gruppe 1,4-Bicyclo[2,2,2]-octylen-, spiro[3.3]heptan-2,6-diyl, Naphthalin-2,6-diyl, Decahydronaphthalin-2,6-diyl, 1,2,3,4-Tetrahydro-naphthalin-2,6-diyl, Thiophen-2,5-diyl, wobei die Gruppen a), b) und c) ein oder mehrfach durch eine Gruppe L substituiert sein können,
L bei jedem Auftreten unabhängig F, Cl, CN, SCN, SF₅ oder geradkettiges oder verzweigtes, jeweils optional fluoriertes, Alkyl, Alkoxy, Alkylcarbonyl, Alkoxycarbonyl, Alkylcarbonyloxy oder Alkoxycarbonyloxy mit 1 bis 12 C-Atomen,
Z¹ und Z² jeweils unabhängig voneinander eine Einfachbindung, -CF₂O-, -OCF₂-, -CF₂S-, -SCF₂-, -CH₂CH₂-, -CF₂CF₂-, -CF₂CH₂-, -CH₂CF₂-, -CHF-CHF-, -C(O)O-, -OC(O)-, -CH₂O-, -OCH₂-, -CH₂S-, -SCH₂-, -CF=CH-, -CH=CF-, -CF=CF-, -CH=CH- oder -C≡C- ,
n 0 bedeuten.

2. Verbindung der Formel I gemäß Anspruch 1, dadurch gekennzeichnent, daß W -S- bedeutet.

3. Verbindung der Formel I gemäß einem oder mehreren der Ansprüche 3. 1 oder 2, dadurch gekennzeichent, daß mindestens einer der Reste Y¹ und Y² von H verschieden ist.

4. Verbindung der Formel I gemäß einem oder mehreren der Ansprüche 1 bis 3, dadurch gekennzeichent, daß Y¹ und Y² beide F bedeuten.

5. Verbindung der Formel I gemäß Anspruch 1, **dadurch gekennzeichnet, daß** die Verbindung ausgewählt ist aus der Gruppe der Verbindungen der Formeln I-1 und I-2 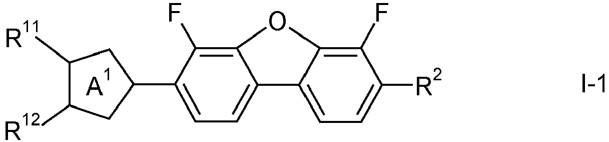 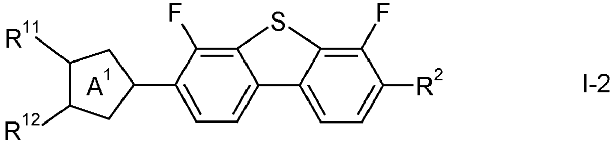 wobei die auftretenden Gruppen die in Anspruch 1 angegebenen Bedeutungen haben.

6. Verbindung gemäß einem oder mehreren der Ansprüche 1 bis 5, **dadurch gekennzeichnet, daß** die Gruppe 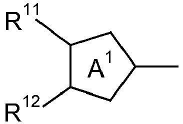 für 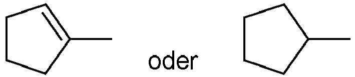 steht.

7. Verbindung gemäß einem oder mehreren der Ansprüche 1 bis 6, **dadurch gekennzeichnet, daß**
R² Methyl, Ethyl, n-Propyl, n-Butyl, n-Pentyl, n-Hexyl oder n-Heptyl bedeutet.

8. Verbindung gemäß einem oder mehreren der Ansprüche 1 bis 6, **dadurch gekennzeichnet, daß**
R² Methoxy, Ethoxy, n-Propoxy, n-Butoxy, n-Pentoxy, n-Hexoxy oder n-Heptoxy bedeutet.

9. Verbindung gemäß einem oder mehreren der Ansprüche 1 bis 6, **dadurch gekennzeichnet, daß**
R² F, Cl, CN, SCN, SF₅, CF₂H, CF₃, OCF₂H, OCF₃, oder -OCH=CF₂ bedeutet.

10. Verbindung der Formel II 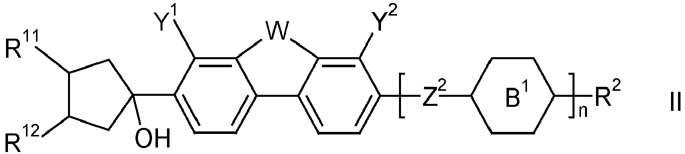 worin R¹¹, R¹², Y¹, Y², W, Z², B¹, R² und n die für Formel I in Anspruch 1 angegebenen Bedeutungen haben.

11. Verfahren zur Herstellung von Verbindungen der Formel I gemäß Anspruch 1, **dadurch gekennzeichnet, daß** eine Verbindung der Formel II gemäß Anspruch 0 durch Eliminierung von Wasser zu einer Verbindung der Formel I umgesetzt wird.

12. Verwendung einer Verbindung nach einem oder mehreren der Ansprüche 1 bis 9 in einem flüssigkristallinen Medium.

13. Flüssigkristallines Medium, **dadurch gekennzeichnet, daß** es eine oder mehrere Verbindungen nach einem oder mehreren der Ansprüche 1 bis 9 enthält.

14. Elektrooptisches Anzeigeelement, **dadurch gekennzeichnet, daß** es ein flüssigkristallines Medium nach Anspruch 13 enthält. Die Erfindung betrifft Verbindungen der Formel I 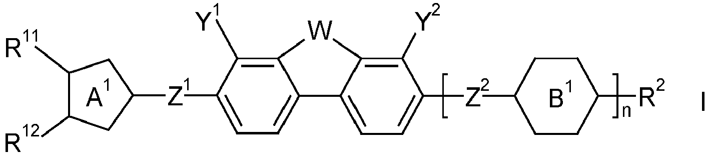 worin auftretenden Gruppen und Parameter die in Anspruch 1 angegebenen Bedeutungen haben, ihre Herstellung, ihre Verwendung als Komponenten in flüssigkristallinen Medien sowie elektrooptische Anzeigeelemente, die die erfindungsgemäßen flüssigkristallinen Medien enthalten.

## Claims

1. Compound of the formula I 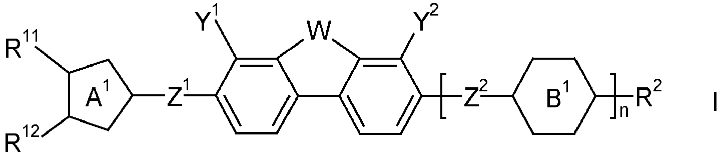 in which
W denotes -O- or -S-,
Y¹ and Y², independently of one another, denote H, F, CI, CN or CF₃,
R¹¹ and R¹², independently of one another, denote H, an alkyl or alkenyl radical having up to 15 C atoms which is unsubstituted, monosubstituted by CN or CF₃ or at least monosubstituted by halogen, where, in addition, one or more CH₂ groups in these radicals may be replaced by -O-, -S-, -C=C-, -CF₂O-, -OCF₂-, -OC-O- or -O-CO- in such a way that O atoms are not linked directly to one another,
R² denotes an alkyl or alkenyl radical having up to 15 C atoms which is unsubstituted, monosubstituted by CN or CF₃ or at least monosubstituted by halogen, where, in addition, one or more CH₂ groups in these radicals may be replaced by -O-, -S-, -C=C-, -CF₂O-, -OCF₂-, -OC-O- or -O-CO- in such a way that O atoms are not linked directly to one another, furthermore H, halogen, CN, SCN or SF₅,
A¹ denotes a 1,3-cyclopentylene radical, which may be monounsaturated and which may be mono- or polysubstituted by a group L,
B¹ on each occurrence, identically or differently, denotes,
a) a 1,4-phenylene radical, in which one or two CH groups may be replaced by N,
b) a 1,4-cyclohexenylene or 1,4-cyclohexylene radical, in which one or two non-adjacent CH₂ groups may be replaced by -O- or -S-, or a cyclobutane-1,3-diyl radical,
c) a radical from the group 1,4-bicyclo[2.2.2]-octylene, spiro[3.3]heptane-2,6-diyl, naphthalene-2,6-diyl, decahydronaphthalene-2,6-diyl, 1,2,3,4-tetrahydro-naphthalene-2,6-diyl, thiophene-2,5-diyl, where the groups a), b) and c) may be mono- or polysubstituted by a group L,
L on each occurrence, independently, denotes F, CI, CN, SCN, SF₅ or straight-chain or branched, in each case optionally fluorinated alkyl, alkoxy, alkylcarbonyl, alkoxy-carbonyl, alkylcarbonyloxy or alkoxycarbonyloxy having 1 to 12 C atoms,
Z¹ and Z² in each case, independently of one another, denote a single bond, -CF₂O-, -OCF₂-, -CF₂S-, -SCF₂-, -CH₂CH₂-, -CF₂CF₂-, -CF₂CH₂-, -CH₂CF₂-, -CHF-CHF-, -C(O)O-, -OC(O)-, -CH₂O-, -OCH₂-, -CH₂S-, -SCH₂-, -CF=CH-, -CH=CF-, -CF=CF-, -CH=CH- or -C≡C- ,
n denotes 0.

2. Compound of the formula I according to Claim 1, **characterised in that** W denotes -S-.

3. Compound of the formula I according to one or more of Claims 1 or 2, **characterised in that** at least one of the radicals Y¹ and Y² is other than H.

4. Compound of the formula I according to one or more of Claims 1 to 3, **characterised in that** Y¹ and Y² both denote F.

5. Compound of the formula I according to Claim 1, **characterised in that** the compound is selected from the group of the compounds of the formulae I-1 and I-2 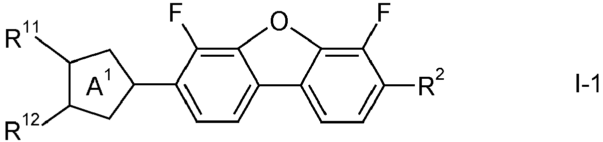 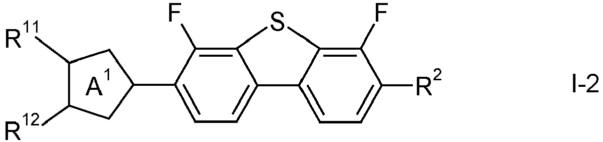 where the groups occurring have the meanings indicated in Claim 1.

6. Compound according to one or more of Claims 1 to 5, **characterised in that** the group 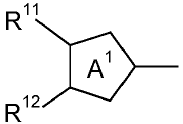 stands for

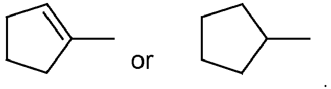

7. Compound according to one or more of Claims 1 to 6, **characterised in that**
R² denotes methyl, ethyl, n-propyl, n-butyl, n-pentyl, n-hexyl or n-heptyl.

8. Compound according to one or more of Claims 1 to 6, **characterised in that**
R² denotes methoxy, ethoxy, n-propoxy, n-butoxy, n-pentoxy, n-hexoxy or n-heptoxy.

9. Compound according to one or more of Claims 1 to 6, **characterised in that**
R² denotes F, CI, CN, SCN, SF₅, CF₂H, CF₃, OCF₂H, OCF₃ or -OCH=CF₂.

10. Compound of the formula II 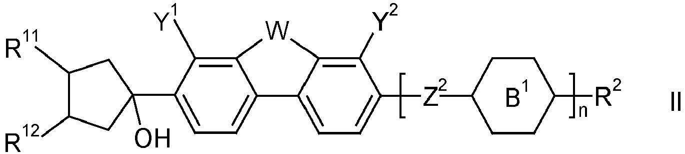 in which R¹¹, R¹², Y¹, Y², W, Z², B¹, R² and n have the meanings indicated for formula I in Claim 1.

11. Process for the preparation of compounds of the formula I according to Claim 1, **characterised in that** a compound of the formula II according to Claim 10 is converted into a compound of the formula I by elimination of water.

12. Use of a compound according to one or more of Claims 1 to 9 in a liquid-crystalline medium.

13. Liquid-crystalline medium, **characterised in that** it comprises one or more compounds according to one or more of Claims 1 to 9.

14. Electro-optical display element, **characterised in that** it contains a liquid-crystalline medium according to Claim 13.

## Revendications

1. Composé de formule I 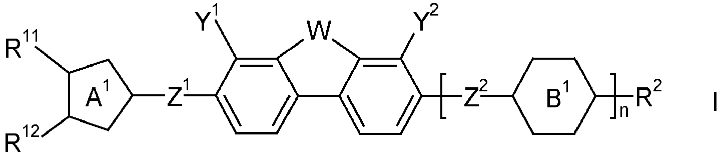 dans laquelle
W désigne -O- ou -S-,
Y¹ et Y², indépendamment l'un de l'autre, désignent H, F, Cl, CN ou CF₃,
R¹¹ et R¹², indépendamment l'un de l'autre, désignent H, un radical alkyle ou alcényle ayant jusqu'à 15 atomes de C qui est non substitué, monosubstitué par CN ou CF₃ ou au moins monosubstitué par halogène, où, de plus, un ou plusieurs groupements CH₂ dans ces radicaux peuvent être remplacés par -O-, -S-, -C=C-, -CF₂O-, -OCF₂-, -OC-O- ou -O-CO- de telle sorte que les atomes de O ne soient pas liés directement les uns aux autres,
R² désigne un radical alkyle ou alcényle ayant jusqu'à 15 atomes de C qui est non substitué, monosubstitué par CN ou CF₃ ou au moins monosubstitué par halogène, où, de plus, un ou plusieurs groupements CH₂ dans ces radicaux peuvent être remplacés par -O-, -S-, -C=C-, -CF₂O-, -OCF₂-, -OC-O- ou -O-CO- de telle sorte que les atomes de O ne soient pas liés directement les uns aux autres, en outre H, halogène, CN, SCN ou SF₅,
A¹ désigne un radical 1,3-cyclopentylène, qui peut être monoinsaturé et qui peut être mono- ou polysubstitué par un groupement L,
B¹ à chaque occurrence, de manière identique ou différente, désigne,
a) un radical 1,4-phenylène, dans lequel un ou deux groupements CH peuvent être remplacés par N,
b) un radical 1,4-cyclohexénylène ou 1,4-cyclohexylène, dans lequel un ou deux groupements CH₂ non adjacents peuvent être remplacés par -O- ou -S-, ou un radical cyclobutane-1,3-diyle,
c) un radical choisi dans le groupe constitué par 1,4-bicyclo[2.2.2]-octylène, spiro[3.3]heptane-2,6-diyle, naphtalène-2,6-diyle, décahydronaphtalène-2,6-diyle, 1,2,3,4-tétrahydro-naphtalène-2,6-diyle, thiophène-2,5-diyle,
où les groupements a), b) et c) peuvent être mono- ou polysubstitués par un groupement L,
L à chaque occurrence, indépendamment, désigne F, Cl, CN, SCN, SF₅ ou alkyle, alcoxy, alkylcarbonyle, alcoxy-carbonyle, alkylcarbonyloxy ou alcoxycarbonyloxy à chaîne linéaire ou ramifiée, dans chaque cas éventuellement fluoré, ayant de 1 à 12 atomes de C,
Z¹ et Z² dans chaque cas, indépendamment l'un de l'autre, désignent une liaison simple, -CF₂O-, -OCF₂-, -CF₂S-, -SCF₂-, -CH₂CH₂-, -CF₂CF₂-, -CF₂CH₂-, -CH₂CF₂-, -CHF-CHF-, -C(O)O-, -OC(O)-, -CH₂O-, -OCH₂-, -CH₂S-, -SCH₂-, -CF=CH-, -CH=CF-, -CF=CF-, -CH=CH- ou -C≡C-,
n désigne 0.

2. Composé de formule I selon la revendication 1, **caractérisé en ce que** W désigne -S-.

3. Composé de formule I selon l'une ou plusieurs parmi les revendications 1 ou 2, **caractérisé en ce qu'**au moins l'un des radicaux Y¹ et Y² est autre que H.

4. Composé de formule I selon l'une ou plusieurs parmi les revendications 1 à 3, **caractérisé en ce que** Y¹ et Y² désignent tous deux F.

5. Composé de formule I selon la revendication 1, **caractérisé en ce que** le composé est choisi dans le groupe constitué par les composés de formules I-1 et I-2 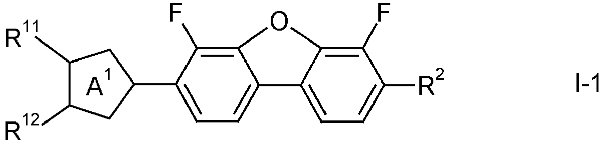 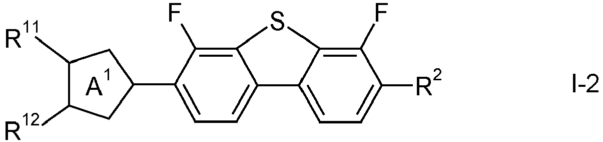 où les groupements présents revêtent les significations indiquées selon la revendication 1.

6. Composé selon l'une ou plusieurs parmi les revendications 1 à 5, **caractérisé en ce que** le groupement 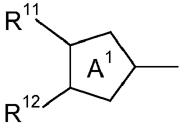 représente

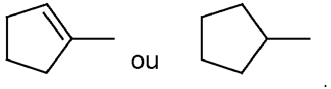

7. Composé selon l'une ou plusieurs parmi les revendications 1 à 6, **caractérisé en ce que**
R² désigne méthyle, éthyle, n-propyle, n-butyle, n-pentyle, n-hexyle ou n-heptyle.

8. Composé selon l'une ou plusieurs parmi les revendications 1 à 6, **caractérisé en ce que**
R² désigne méthoxy, éthoxy, n-propoxy, n-butoxy, n-pentoxy, n-hexoxy ou n-heptoxy.

9. Composé selon l'une ou plusieurs parmi les revendications 1 à 6, **caractérisé en ce que**
R² désigne F, Cl, CN, SCN, SF₅, CF₂H, CF₃, OCF₂H, OCF₃ ou -OCH=CF₂.

10. Composé de formule II 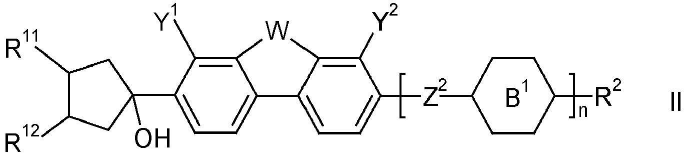 dans laquelle R¹¹, R¹², Y¹, Y², W, Z², B¹, R² et n revêtent les significations indiquées pour la formule I selon la revendication 1.

11. Procédé de préparation de composés de formule I selon la revendication 1, **caractérisé en ce qu'**un composé de formule II selon la revendication 10 est converti en un composé de formule I par élimination d'eau.

12. Utilisation d'un composé selon l'une ou plusieurs parmi les revendications 1 à 9, dans un milieu cristallin liquide.

13. Milieu cristallin liquide, **caractérisé en ce qu'**il comprend un ou plusieurs composés selon l'une ou plusieurs parmi les revendications 1 à 9.

14. Elément d'affichage électro-optique, **caractérisé en ce qu'**il contient un milieu cristallin liquide selon la revendication 13.
